(19) Europäisches Patentamt
European Patent Office
Office européen des brevets

(11) EP 0 483 304 B1

(12) EUROPEAN PATENT SPECIFICATION

(45) Date of publication and mention of the grant of the patent:
**19.02.2003 Bulletin 2003/08**

(21) Application number: **91907810.5**

(22) Date of filing: **16.04.1991**

(51) Int Cl.[7]: **A61K 35/14**, A61L 2/00

(86) International application number:
**PCT/US91/02504**

(87) International publication number:
**WO 91/016060 (31.10.1991 Gazette 1991/25)**

# (54) METHOD OF INACTIVATION OF VIRAL AND BACTERIAL BLOOD CONTAMINANTS

METHODE ZUR UNSCHÄDLICHMACHUNG VIRALER UND BAKTERIELLER BLUTVERUNREINIGUNGEN

PROCEDE POUR RENDRE INACTIFS DES CONTAMINANTS SANGUINS VIRAUX ET BACTERIENS

(84) Designated Contracting States:
**AT BE CH DE DK ES FR GB GR IT LI LU NL SE**

(30) Priority: **16.04.1990 US 510234**
**20.12.1990 US 632277**
**15.02.1991 US 656254**

(43) Date of publication of application:
**06.05.1992 Bulletin 1992/19**

(73) Proprietor: **BAXTER INTERNATIONAL INC.**
**Deerfield, Illinois 60015 (US)**

(72) Inventors:
- **HACKETT, Roger**
  **Pasadena, CA 91107 (US)**
- **GOODRICH, Raymond, P., Jr.**
  **Pasadena, CA 91106 (US)**
- **VAN BORSSUM WAALKES, Marjan**
  **NL-3906 ZK Veenendaal (NL)**
- **WONG, Victoria, A.**
  **Pasadena, CA 91107 (US)**

(74) Representative:
**Beresford, Keith Denis Lewis et al**
**BERESFORD & Co.**
**2-5 Warwick Court,**
**High Holborn**
**London WC1R 5DH (GB)**

(56) References cited:

| | |
|---|---|
| EP-A- 0 083 999 | EP-A- 0 124 363 |
| EP-A- 0 196 515 | EP-A- 0 334 679 |
| WO-A-90/03187 | US-A- 4 071 412 |
| US-A- 4 409 105 | US-A- 4 620 908 |
| US-A- 4 684 521 | US-A- 4 874 690 |
| US-A- 4 878 891 | US-A- 4 950 665 |

- **Vox Sang, Vol. 26, issued 1974 K. GANSHIRT et al., "A five-bag system for washing fresh and frozen erythrocytes and their preservation", pages 66-73, see page 66.**
- **Cryobiology, Vol. 10, issued 1973, D. PRIBOR, "Studies with Dextran 40 in cryopreservation of blood", pages 93-103, see entire article.**
- **Acta Vet Scand, Vol. 20, issued 1979, V. MYHRVOID, "Cryopreservation of sheep red blood cells", pages 531-536, see entire article.**
- **Mutation Research, Vol. 81, issued 1981, W. FIRTH et al., "Azido Analogs of Acridine: PHOTOAFFINITY PROBES FOR FRAMESHIFT MUTAGENESIS IN SALMONELLA typhimurium", pages 295-309, see page 299.**
- **R. ACHESON et al., "ACRIDINES" published 1956 by Interscience Publishers, Inc. (N.Y.), pages 339-361, see pages 352-355.**
- **VOX SANG., Vol. 55, issued 1988, G. ESPERSEN et al., "IRRADIATED BLOOD PLATELET CONCENTRATES STORES FOR FIVE DAYS - EVALUATION BY IN VITRO TESTS", pages 218-221, see Abstract.**

EP 0 483 304 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **EXP. PARASITOL., Vol. 31, issued 1972, C. LANTZ et al., "PLASMODIUM berglei: INHIBITED INCORPORATION OF AMP-8-3H into NUCLEIC ACIDS OF ERYTHROCYTE-FREE MALARIAL PARASITES BY ACRIDINES, PHENANTHRIDINES, AND 8-AMINOQUINOLINES", pages 255-261, see page 258.**
- **PHARM. DELT., EPISTOM. EKDOSIS, Vol. 1, No. 2, issued 1971, J. POLAK et al. "THE BACTERICIDAL AND FUNGI-CIDAL ACTIVITY OF SOME QUINOLINIUM COMPOUNDS", pages 27-33, see entire article.**

## Description

### FIELD OF THE INVENTION

This invention relates to the general field of biochemistry and medical sciences, and specifically to inactivating viral/bacterial contamination of lyophilized or reconstituted blood cell compositions comprising erythrocytes, platelets, etc, or protein fractions.

### BACKGROUND OF THE INVENTION

A major concern in the use of stored or donated homologous blood or plasma protein preparations derived from human blood is the possibility of viral and bacterial contamination.

Viral inactivation by stringent sterilization is not acceptable since this could also destroy the functional components of the blood, particularly the erythrocytes (red blood cells) and the labile plasma proteins. Viable RBC's can be characterized by one or more of the following: capability of synthesizing ATP; cell morphology; $P_{50}$ values; oxyhemoglobin, methemoglobin and hemichrome values; MCV, MCH, and MCHC values; cell enzyme activity; and in vivo survival. Thus, if lyophilized then reconstituted and virally inactivated cells are damaged to the extent that the cells are not capable of metabolizing or synthesizing ATP, or the cell circulation is compromised, then their utility in transfusion medicine is compromised.

There is an immediate need to develop protocols for the deactivation of viruses that can be present in the human red blood supply. For example, only recently has a test been developed for Non A, Non B hepatitis, but such screening methods, while reducing the incidence of viral transmission, do not make the blood supply completely safe or virus free. Current statistics indicate that the transfusion risk per unit of transfused blood is as high as 1:100 for Non A, Non B hepatitis, and ranges from 1:40,000 to 1:1,000,000 for HIV, depending on geographic location. Clearly, it is desirable to develop a method which inactivates or removes virus indiscriminately from the blood.

Contamination problems also exist for blood plasma protein fractions, such as plasma fractions containing immune globulins and clotting factors. For example, new cases of non A, non B hepatitis have occurred in hemophilia patients receiving protein fractions containing Factor VIII which have been treated for viral inactivation according to approved methods. Therefore, there is a need for improved viral inactivation treatment of blood protein fractions.

International Patent Application No. WO90/03187 is concerned with destroying tumour or other pathogenic biological contaminants infecting animal body tissues using a pre-activated therapeutic agent derived from a photoactive compound such as an acridine derivative.

European Patent Application No. 0196515 discloses a method of viral decontamination of protein compositions by exposing the compositions to light in the presence of a photosensitizer to effect inactivation of contaminant viruses. The photosensitizer is preferentially adsorbed to nucleic acids.

European Patent Application No. 0124363 is another document dealing with photochemical decontamination, this time directed to whole blood or blood components. Preferential uptake of the photochemical sensitizer in nucleic acids is also taught in this document.

United States Patent No. 4620908 teaches destruction of microbial contamination in protein materials using gamma radiation at low temperature.

European Patent Application No. 0334679 also teaches inactivation of infectious agents by means of irradiation at low temperature.

It is an object of the present invention to provide a method for the inactivation of viral and bacterial contaminants present in blood and blood protein fractions.

### SUMMARY OF THE INVENTION

The present invention provides a method for viral/bacterial inactivation of dried or reconstituted cells (erythrocytes, platelets, hemosomes and other cellular or cell-like components) or blood protein fractions, which allows for the cells or protein fractions to be useful in a transfusable state, while still maintaining relatively high cell viability, ATP synthesis and oxygen transport, in the case of cellular components, and therapeutic efficacy, in the case of protein fractions.

According to the invention, there is provided a process for reducing viral and/or bacterial contamination in a composition comprising red blood cells, platelets and/or proteins, said process comprising the steps of contacting said composition with at least one chemical sensitizer selected from the group consisting of compounds which bind to DNA and/or RNA, and exposing said composition to radiation of sufficient wavelength and intensity for a period of time sufficient to cause said sensitizer to further reduce viral and bacterial contamination in said composition; characterised by lyophilizing the composition prior to said step of exposing said composition to radiation.

The lyophilization and reconstitution media may be utilized to lyophilize and reconstitute proteins, particularly, blood plasma protein fractions. The protein fraction may be virally/bacterially deactivated by mixing with a chemical sensitizer, lyophilized (freeze-dried), then irradiated. When the lyophilization media is used, it is contemplated that the constituents of the media also serve to provide some degree of protection of the dry proteins during irradiation.

A preferred embodiment comprises reducing viral and bacterial contamination of dried or reconstituted cells with washing solutions containing a polymer or mixture of polymers having a molecular weight in the range of about 1K to 360K, followed by one or more additional wash cycles using a wash of a dextrose-saline solution at a pH in the range of about 7.0-7.4. The dextrose-saline solution will also contain a polymer having a molecular weight in the range of about 1K to 40K, and preferably about 2.5K.

The composition of reconstituted cells will also preferably contain a monosaccharide.

Preferably the cells will have been previously lyophilized using a lyophilization solution buffered in the range of pH of 7.0 to 7.4 preferably by a phosphate-buffered solution. A typical phosphate-buffered lyophilization solution will comprise mono-and di-basic potassium and sodium phosphate (usually in the range of 1-10 mM each) and 5-10 mM adenine. This solution maintains the pH at around 7.2.

A preferred phosphate-buffered solution to be used as the lyophilization buffer will comprise nicotinic acid, reduced glutathione, glutamine, inosine, adenine, monopotassium phosphate, magnesium chloride disodium phosphate all of which will serve as a basic salt buffer at a pH of about 7.2. In addition this lyophilization buffer will contain a final concentration of about 26% weight by volume of a monosaccharide, preferably 1.7 M glucose, and a final concentration of about 3.0% weight by volume of polyvinylpyrrolidone (average molecular weight of 360K), and a final concentration of about 15% weight by volume of hydroxyethyl starch (average molecular weight of 500K).

The term lyophilization is broadly defined as freezing a substance and then reducing the concentration of the solvent, namely water, by sublimation and desorption, to levels which will no longer support biological or chemical reactions. Usually, the drying step is accomplished in a high vacuum. However, with respect to the storage of cells and particularly erythrocytes, the extent of drying (the amount of residual moisture) is of critical importance in the ability of cells to withstand long-term storage at room temperature. Using the procedure described herein, cells may be lyophilized to a residual water content of less than 10 weight %, preferably less than 3%, and still be reconstituted to transfusable, therapeutically useful cells. Cells with about 3 weight % water content using this procedure may be stored for up to two weeks at room temperature, and at 4°C for longer than eight months, without decomposition. This far exceeds the current A.A.B.B. standard for refrigerated storage of red blood cells of six weeks at 4°C or less than one day at room temperature without decomposition. These dried cells may be deactivated using a chemical sensitizer described herein.

According to the preferred embodiment of the present invention the washed packed red blood cells are mixed with a chemical sensitizer, then washed to remove excess sensitizer not bound to viral or bacterial nucleic acid, and the treated cells are then lyophilized. The dry cell and sensitizer mixture will then be irradiated, typically with gamma radiation, at an intensity of about 3K-50K rads, for a period of time sufficient to destroy viruses (in particular, the single-stranded or double-stranded RNA/DNA viruses), without any substantial adverse effect on the recovery and usefulness of the cells. Other wavelengths of electromagnetic radiation such as X-rays, may be used.

In another preferred embodiment, the chemical sensitizers may be added to liquid protein preparations, then lyophilized and irradiated. Particularly preferred are blood protein preparations, including but not limited to, plasma proteins, blood protein extracts, clotting factors (such as Factors VIII and IX), immune globulins and serum albumin.

Dry (lyophilized) cells or protein fractions may be directly mixed with the chemical sensitizer, then irradiated.

From the foregoing description, it will be realized that the invention can be used to selectively bind a metal atom or a metal atom containing chemical sensitizer to blood-transmitted viruses, bacteria, or parasites. Also monoclonal or polyclonal antibodies directed against specific viral antigens (either coat proteins or envelope proteins) may be covalently coupled with either a metal atom or a metal atom-containing sensitizer compound, thereby increasing the effective cross-section of the contaminant to penetrating or other forms of radiation energy.

Since cell compositions also comprise a variety of proteins, the method of decontamination of cells described herein is also applicable to protein fractions, particularly blood plasma protein fractions, including, but not limited to, fractions containing clotting factors (such as Factor VIII and Factor IX), serum albumin and/or immune globulins. The viral and bacterial inactivation may be accomplished by treating a protein fraction with a sensitizer as described herein. A protein fraction which has been lyophilized and reconstituted may be sensitized and irradiated to deactivate possible contamination. It is contemplated that liquid and frozen protein fractions may also be decontaminated according to the present invention.

Depending upon the nature of the presumed radiolytic mechanism of the sensitizer reaction with the virus, other types of radiation may be used, such as X-ray, provided the intensity and power utilized is sufficient to inactivate the viral contamination without adverse effect on the cells. Mature human red blood cells and platelets lack nucleic acids, therefore the nucleic acid binding sensitizers selectively target contaminating viruses and bacteria. Although described in connection with viruses, it will be understood that the methods of the present invention are generally also

useful to any biological contaminant found in stored blood or blood products, including bacteria and blood-transmitted parasites.

DETAILED DESCRIPTION OF THE INVENTION

The cells are preferably prepared by immersing a plurality of erythrocytes, platelets and/or hemosomes, etc. in a physiologic buffered aqueous solution containing a carbohydrate, and one or more biologically compatible polymers, preferably having amphipathic properties. By the term amphipathic it is meant there are hydrophobic and hydrophilic portions on a single molecule. This immersion is followed by freezing the solution, and drying the frozen solution to yield novel freeze-dried erythrocytes containing less than 10%, and preferably about 3% or less by weight of moisture, which, when reconstituted, produce a significant percentage of viable, transfusably useful red blood cells, platelets or hemosomes. Preferred methods of reconstitution of the lyophilized composition are described below. Although described in connection with red blood cells, it will be understood that the methods are generally also useful to lyophilize platelets, hemosomes, and blood protein fractions.

The carbohydrate utilized to prepare erythrocyte, platelet and/ or hemosome compositions according to the invention is biologically compatible with the erythrocytes, platelets or hemosomes, that is, non-disruptive to the cells or hemosome membrane, and one which permeates, or is capable of permeating, the membrane of the erythrocytes, platelets or hemosomes. It is also advantageous to stabilize proteins, especially labile blood proteins, with the carbohydrates during lyophilization and irradiation according to the invention. The carbohydrate may be selected from the group consisting of monosaccharides, since disaccharides do not appear to permeate the membrane to any significant extent. Monosaccharide pentoses and hexoses are preferred as is a final concentration of from about 7.0 to 37.5 weight % in phosphate buffered saline (PBS) or a phosphate buffered solution, preferably about 26%. Xylose, glucose, ribose, mannose and fructose are employed to particular advantage.

It will be understood that the cells may be lyophilized using other protocols and irradiated as described below. Although viral inactivation will be attained, the advantage of retaining a significant percentage of viable useful red blood cells is lost if the described lyophilization procedure is not followed.

The invention will be hereafter described in connection with erythrocytes (RBC's) but it will be understood it is also applicable to platelets, hemosomes or other blood cell types or biological cells, as well as protein fractions, particularly plasma protein fractions.

The erythrocytes will preferably be prepared from whole blood centrifugation, removal of the plasma supernatant and resuspending the cells in PBS or a phosphate buffered solution or a commercial dextrose-saline solution. This wash cycle may be repeated 2-3 times preferably using a commercial dextrose-saline solution, then the packed cells are diluted with the lyophilization buffer described above so that the final diluted concentration of carbohydrate and polymer are maintained in the necessary ranges.

Alternatively, commercially available packed blood cells may be used, which typically are prepared in CPDA (commercial solution containing citrate, phosphate, dextrose and adenine).

Upon lyophilization to a moisture content of less than 10%, and preferably less than 3%, the lyophilized cells may be maintained under vacuum in vacuum-tight containers, or under nitrogen or other inert gas, at room temperatures for extended periods of time in absence of or without significant degradation of their desirable properties when reconstituted for use as transfusable cells. In using the preferred lyophilization method disclosed herein, a particular advantage of the present invention is that the lyophilized cells may be stored at room temperature for extended periods of time, thus obviating the need for low temperature refrigeration which is required for storing liquid CPDA preserved red blood cells prepared by methods of the prior art. The present invention also obviates the need for very low temperature (-80°C) frozen storage of red blood cells in glycerol.

By using the preferred reconstitution method disclosed herein it is a further advantage that the lyophilized red blood cells may be reconstituted at normal temperatures, i.e. greater than about 4°C up to about 37°C, which corresponds to normal human body temperature, and preferably at room temperature (about 22°C). The reconstitution medium is preferably a solution comprising a polymer or mixture of polymers having a molecular weight of from about 2.5K to 360 K, preferably 5K to about 360K, present in a concentration in the range of about 12 to 30% weight by volume. This polymer may be the same polymer utilized to lyophilize the red blood cells as described above. Hence the polymers polyvinylpyrrolidone, hydroxyethyl starch, and dextran are particularly preferred and most preferred is polyvinylpyrrolidone (preferably molecular weight about 10K) present in a concentration of about 19% weight by volume in the reconstitution solution. The reconstitution solution will be buffered again typically by phosphate-buffered solution comprising monopotassium phosphate and disodium phosphate as described hereinabove to maintain a pH within the range of about 7.0 to 7.4. The most particularly preferred polymer is polyvinylpyrrolidone of an average molecular weight of about 10K. The most preferred reconstitution buffer will also contain adenosine triphosphate (ATP) in a final concentration of about 5mM.

The polymers may be present in the various solutions from a final concentration of about 3.6K weight % up

to saturation, and have a molecular weight in the range of from about 2.5K to about 360K. Preferably, the polymers have molecular weights in the range of from about 2.5K to about 500K, most preferably from about 2.5K to 50K, and are present in a concentration of from about 3.6 weight % up to the limit of solubility of the polymer in the solution. Polymers selected from the group consisting of polyvinylpyrrolidone (PVP) and polyvinylpyrrolidone derivatives, and dextran and dextran derivatives provide significant advantages. Most preferred is the use of polyvinylpyrrolidone (an amphipathic polymer) of average molecular weight in the range of 2.5-360K in an amount in the range of 3-20% weight by volume in the solution prior to lyophilization. Amino acid based polymers (i.e., proteins), dextrans or hydroxyethyl starch may also be employed. In the lyophilization buffer hydroxyethyl starch (M-HES) with an average molecular weight of about 500K is employed in a 15% weight by volume final concentration. Other amphipathic polymers may be used, such as poloxamers in any of their various forms. The use of the carbohydrate-polymer solution in the lyophilization of red blood cells allows for the recovery of intact cells, a significant percentage of which contain biologically-active hemoglobin.

The most preferred reconstitution buffer will be a solution comprising monopotassium phosphate, disodium phosphate and ATP, all of which form a basic salt buffer at a pH of about 7.2, which also contains about 19% weight by volume of polyvinylpyrrolidone (average molecular weight about 10K).

The reconstitution solution may also optionally contain a monosaccharide, preferably present in the concentration range of about 7.0 to 37.5% weight by volume. The preferred monosaccharides are xylose, glucose, ribose, mannose and fructose.

In the most preferred embodiment, the lyophilized erythrocytes can be reconstituted by mixing with an equal volume of the reconstitution buffer at a temperature of about 37°C and mixed. By "equal" it is meant that the volume is the same as the starting volume prior to lyophilization. After initial reconstitution, the solution is preferably diluted 1: 1 with 1-4 additional volumes of the reconstitution buffer at a temperature of about 37°C with added mixing until fully hydrated.

Then, it is preferred that the rehydrated cells be washed according to the following procedure. It is realized, however, that once the cells are reconstituted with reconstitution buffer they are in a hydrated and useful form, but the combination of washings described hereinafter are preferred, specifically for clinical purposes.

After separating the cells from the reconstitution buffer by centrifugation, the resulting packed cells are preferably resuspended at room temperature in (approximately the volume used in the initial reconstitution) a wash buffer comprising nicotinic acid, inosine, adenine, glutamine, and magnesium chloride, all present at about 0.4-10mM further comprising sodium chloride and potassium chloride each at about 30mM, buffered by 10mM disodium phosphate to pH 7.2. This wash buffer further comprises a monosaccharide, preferably glucose at a concentration of about 20mM, and a polymer, preferably polyvinylpyrroltidone, of a molecular weight 40K and present at a concentration of about 16% weight by volume. Separation by centrifugation completes the first post-rehydration step, a washing step.

After the washing step the rehydrated cells may be suspended in a dextrose-saline transfusion buffer at room temperature which preferably contains polyvinylpyrrolidone at a 10% weight by volume final concentration, with an average 2.5K molecular weight. The cells can be used as is or be returned to autologous plasma. Additional wash steps in a phosphate-buffered diluent buffer can further remove viruses, but this step is optional for preparation of rehydrated, transfusible cells.

The reconstitution and washings described above will in most instances achieve about 4 log reduction of any viral and bacterial contamination, where 1 log reduction is achieved by drying and 3 log reduction is achieved by washing. Of course, different viruses may respond differently, potentially resulting in more than 4 log reduction of contamination.

The reconstituted cells have characteristics which render them transfusable and useful for therapeutic purposes in that their properties are similar to that of fresh (i.e. not previously lyophilized) red blood cells. Typically reconstituted red blood cells according to the present invention have an oxyhemoglobin content greater than about 90% of that in normal red blood cells. Hemoglobin recovery prior to any washing step is typically in the range of 80 to 85%. The overall cellular hemoglobin recovery including the post-hydration washing steps is about 20 to 30%. The morphology of the reconstituted cells according to the present invention (by scanning electron microscope) typically shows no holes or gaps, and primarily discocytic with some stomatocytic morphology. The oxygen carrying capacity of fresh red blood cells (as measured by $P_{50}$, the oxygen partial pressure at which 50% of the oxygen molecules are bound) was measured to be in the range of about 26 to 28 (average 26.7); with an average Hill coefficient (a measure of the cooperative binding of oxygen molecules to native hemoglobin) of 1.95. The typical $P_{50}$ for erythrocytes lyophilized and reconstituted according to the present invention is about 27.5 (average) with an average Hill coefficient of 2.08. Assays of ATP in the reconstituted cells indicate ATP levels suggesting normal ATP to ADP metabolism. Normal hemagglutination by readily available blood typing antisera of red blood cells made according to the present invention is also typically found.

This lyophilization and reconstitution procedure advantageously and significantly diminishes viral/bacterial contamination in cell-like material (such as hemosomes), and protein fractions. The contamination can be further reduced by the radiation sensitizing and treatment, particularly while the cells or protein fractions are in the dry state.

The starting packed red blood cells or proteins (which may initially be in a liquid or lyophilized state) are mixed with a sufficient amount (based on total wet weight of cells) of a chemical sensitizer. Preferably, in a composition of packed red blood cells (about 10% hematocrit) about 0.1 to 1 mg of the chemical sensitizer will be used per ml of packed cells. Preferably, the mixture will be irradiated with gamma radiation in the range of 3K-50K rads, typically about 3K rads. Preferred exposure is from 1-10 minutes, if using gamma radiation. Alternatively, UV light (320 nm) may be used, particularly for protein fractions. Preferred exposure is from 1-10 minutes, preferably 3 minutes, if using UV radiation. By this irradiation in presence of a sensitizer, there will be about a 6 log reduction of viral and bacterial contamination, based on contamination present prior to washing and irradiation.

The present invention provides a selective method of generating free radicals derived from chemical sensitizers only in the vicinity of viral RNA or DNA. Indiscriminate radiolysis of blood containing virus in a hydrated state produces hydroxyl radical. However, the hydroxyl radical will damage both the red blood cells and associated proteins as well as the viral target. Thus, viral inactivation would be achieved at the sacrifice of red cell viability. Therefore, sensitizers which bind to DNA and/or RNA and which can be selected to generate radicals upon irradiation, are required. Since the radiolysis can be performed in the dry state (preferably less than 10% residual moisture), generation of hydroxyl radicals from water is greatly reduced. In this manner indiscriminate radical damage is further prevented. Exemplary compounds include:

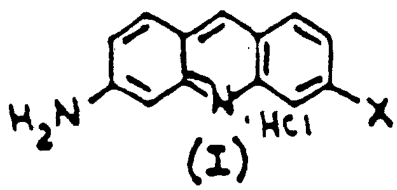

(I)

X = $-N_3$, -I

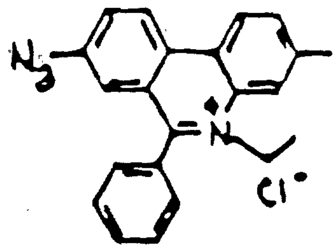

(II)

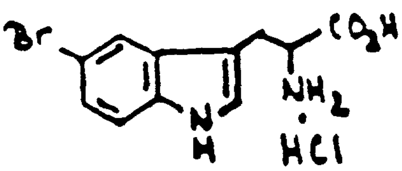

(III)

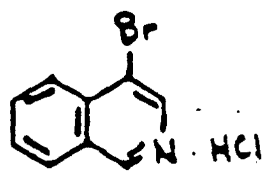

(IV)

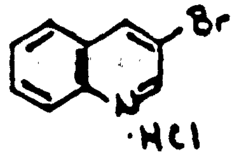

(V)

The preparations of these compounds are known. See Martin, R.F. and Kelly, D.P., Aust. J. Chem., 32, 2637-46 (1979); Firth, W., and Yielding, L.W., J. Org. Chem., 47, 3002 (1982). Other radical-generating reagents which generate radicals upon irradiation are disclosed by Platz et al., Proc. SPIE-Int. Soc. Opt. Eng. 847, 57-60 (1988) and Kanakarajan et al., JACS 110 6536-41 (1988).

The radiation-sensitizing compound (which may also be modified to bear a metal atom substituent) may also be selected from the class consisting of DNA-binding drugs, including, but not limited to, netropsin, BD peptide (a consensus peptide from HMG-1), S2 peptide, and the like. These and other DNA-binding drugs are disclosed in Pjura, P.E., Grzeskowiak, K. and Dickerson, R.E. (1987), J. Mol. Biol. 197, 257-271; and Teng, M., Usman, N., Frederick, C. A. and Wang, A.H.J. (1988), Nucleic Acids Res. 16, 2671-2690.

The radiation sensitizing compound (which may also bear a metal atom) can also comprise a class of DNA-binding proteins and/or polypeptides and/or peptides. Examples of this class of DNA-binding proteins and/or polypeptides and/or peptides are disclosed in Churchill, M.E.A. and Travers, A.A. (1991) Trends in Biochemical Sciences 16, 92-97. Specific examples of DNA-binding peptides include the SE peptide and BD peptide disclosed in the reference herein.

The DNA-binding specificity can be achieved by covalently coupling the radiation sensitizing compound and/or metal atom to either a DNA-binding drug or to a DNA-binding protein or polypeptide or peptide.

VI

Netropsin

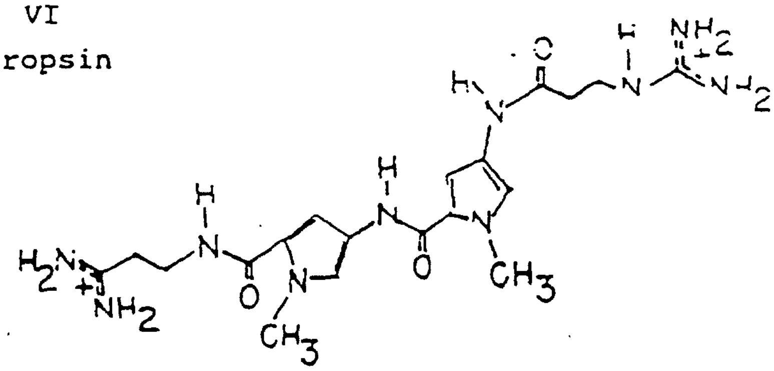

VII

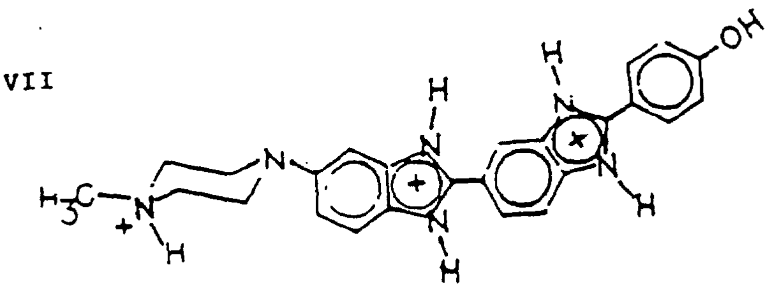

Other sensitizers include specially designed molecules which form triplex DNA, such as those disclosed by Youngquist and Dervan PNAS 82 2565 (1985); Van Dyke and Dervan, Science 225 1122 (1984); Van Dyke and Dervan, Nuc. Acids Res. 11 5555 (1983); Barton and Raphael, PNAS 82 6460 (1985); Barton et al., JACS 106 2172 (1984); and Barton, PNAS 81 1961 (1984). These molecules bind to DNA and RNA, site specifically, if desired, and carry reactive moieties which can generate free radicals in the proximity of the DNA or RNA.

$$R\text{-}I + e^{-} \rightarrow R \bullet I^{-}$$

$$R\text{-}I^{+\bullet} + \text{Guanine} - R\text{-}I + \text{Guanine}^{+\bullet}$$

While not intending to be bound by a theory, it is believed that the ejected electron will be captured by that site with the most favorable electron affinity, which is most likely a second molecule of sensitizer elsewhere in the sample. Electron capture by R-I (or R-Br) leads to dissociation of RX with the formation of a radical. The radical so generated will abstract a C-H hydrogen atom from a sugar moiety of a nearby nucleic acid which in turn will lead to DNA or RNA cleavage and viral inactivation.

The radical cation of the sensitizer ($R\text{-}X^{+\bullet}$) will eventually abstract an electron from that component of the sample with the most favorable oxidation potential. This is most likely guanine. The electron transfer reaction forms guanine radical cation. This substance will react with $O_2$ upon reconstitution with aerated $H_2O$. This process also leads to DNA cleavage and viral inactivation. Unreacted material and reaction by-products will be removed during the washing steps involved in the reconstitution of the lyophilized cells (Table 2). This process will also further remove any virus not inactivated by the treatment described above.

Compounds (1) and (2) bind tightly to DNA and RNA by either intercalation and/or by electrostatic interactions between positively charged ammonium ion groups and the negatively charged phosphate groups of the nucleic acid target. Red blood cells do not contain nucleic acids and accordingly will not bind to such compounds by intercalation.

The best mode for using the invention is to add the sensitizer to potentially contaminated blood solutions, and to expose to gamma radiation or x-rays. Fluid solutions of blood are preferably exposed to 3000 rads, and dried lyophilized solid formulations are preferably exposed to 10,000 rads of radiation. It is known that the red cells will survive

these doses of radiation in the absence of a sensitizer. Lyophilized blood can withstand higher dosage levels of radiation than hydrated blood.

The gamma radiation or x-ray will be absorbed primarily by the heavy atom of the sensitizer, which will be bound to viral DNA or RNA. The radiation will ionize the sensitizer as follows:

$$\text{R-I} + \gamma\text{-Ray} \rightarrow \text{R-I}^{+\bullet} + e^{-}$$

(X-Ray)

In some instances, particularly if the sensitizer and red blood cells are allowed to stand together for more than several minutes, sensitizers may diffuse into the red blood cells prior to lyophilization.

Antioxidants such as glutathione (an excellent hydrogen atom donor) may be added to the preparation to augment the red cell defenses against free radical initiated damage. It will be understood that incorporation of the sensitizer into cells will also allow inactivation of intracellular viruses, especially viruses thought to reside inside white blood cells (most packed red blood cell units contain residual white cells), or intracellular blood parasites, such as malaria parasite which infects red blood cells.

The sensitizers are removed from the reconstituted blood serum or protein fraction by the washing protocol described above for lyophilized cells.

It is preferred that gamma or X-ray radiolysis take place in a dried lyophilized blood (or protein), virus, and sensitizer formulation rather than in a wet, fluid material for several reasons. Firstly, the dry material is less sensitive to radiation and can be exposed to larger doses of γ-rays or other penetrating radiation without damage to red blood cells (Table 1). This increases the extent of radiolysis of the sensitizer. Secondly, sensitizer radicals bound to DNA or RNA in the dry state can not dissociate from the virus due to the lack of diffusion in the solid material. This will force the sensitizer radical to react with viral RNA or DNA. Thirdly, the solid state conditions will enhance hydrogen atom transfer reactions of the sensitizer radical with the viral nucleic acid, perhaps by quantum mechanical tunneling. Fourthly, the reconstitution and washing protocol used with lyophilized blood or protein fraction serves as a means to remove unreacted material or reaction by-products, and further removes any virus not affected by the treatment (Table 2).

Other types of radiation may be used including ionizing radiation in general, such as X-ray radiation. In one embodiment a metal atom may be a substituent on a chemical radiation sensitizer molecule which binds to nucleic acids, thereby targeting the embodiments such as bacteria, parasites and viruses. Metal atom substituents of chemical sensitizers for this purpose include Br, I, Zn, Cl, Ca and F. The X-ray source is preferably a tunable source, so that the radiation may be confined to a narrow wavelength and energy band, if so desired. The tunable feature allows for optimization of energy absorption by the metal atoms, thereby directing the absorbed penetrating radiation energy to the production of radicals by a chemical sensitizer bound to nucleic acid.

The present invention is applicable to contaminants which comprise single or double-stranded nucleic acid chains, including RNA and DNA, and viruses, bacteria or other parasites comprising RNA and/or DNA.

To illustrate the invention, red blood cells were lyophilized as described above, irradiated, and tested for erythrocyte characteristics measured. The results are shown in Table 1. The same procedure was then used, except that the bacteriophage T4 (in dextrose saline) was mixed with the cells and then washed successively with four different wash buffers. The results are shown in Table 2.

Table 1:

| Influence of irradiation on lyophilized reconstituted red blood cells. Doses as high as 20,000-50,000 rads do not affect cells in the dry state according to the parameters assayed after reconstitution and listed below. | | |
|---|---|---|
| Exposure of Lyophilized Cells to Gamma Irradiation | | |
| | *Percentage of Control | |
| **Dosage Level** | **20,000 rads** | **50,000 rads** |
| Hb Recovery | 100 | 99 |
| Oxy Hb | No Change from starting value | No Change from starting value |
| Cell Indices | | |
| MCV | 99 | 98 |
| MCH | 100 | 100 |

* Control cells were non-irradiated, lyophilized reconstituted cells.

Table 1: (continued)

| Influence of irradiation on lyophilized reconstituted red blood cells. Doses as high as 20,000-50,000 rads do not affect cells in the dry state according to the parameters assayed after reconstitution and listed below. | | |
|---|---|---|
| Exposure of Lyophilized Cells to Gamma Irradiation | | |
| | *Percentage of Control | |
| **Dosage Level** | **20,000 rads** | **50,000 rads** |
| MCHC | 100 | 100 |
| Metabolism | | |
| ATP (μmol/g Hb) | 79 | 79 |
| Lactate (μmol/g Hb/Hr) | 86 | 79 |

* Control cells were non-irradiated, lyophilized reconstituted cells.

Table 2:

| Reduction in viral titre as a function of washing of the red cells. The procedure used in reconstituting the lyophilized cells involves several washing steps which also reduce the viral titre. The extent of reduction with each wash decreases until a practical limit is attained. This represents an approximate 4 log reduction in viral titre. | | |
|---|---|---|
| Washing Protocol Reduction of Viral Load in Blood | | |
| **Buffer Wash Step** | **Total Amount of Virus** | **Log Reduction** |
| Experiment 1 (non-lyophilized cells) | | |
| Reconstitution | 7.3 Ox $10^7$ | 0 |
| Wash | 4.80 x $10^4$ | 3.2 |
| Diluent | 2.08 x $10^4$ | 3.5 |
| Transfusion | 3.50 x $10^4$ | 3.3 |
| Experiment 2 (lyophilized cells) | | |
| Lyophilization | 3.68 x 108 | 0 |
| Reconstitution | 2.11 x 107 | 1.2** |
| Wash | 2.38 x 104 | 4.2 |
| Diluent | 2.00 x 104 | 4.3 |
| Transfusion | 4.06 x 104 | 4.0 |

In Experiment 1, the effects of lyophilization on viral reduction are not included. In Experiment 2, these effects are included. The marker virus used in these cases was bacteriophage T4. The extent of reduction was determined using the plaque assay.
**This shows an additional about 1 log reduction of contamination due to the drying step.

EXAMPLE 1

Packed human red blood cells purified from donated whole blood are washed free of the anticoagulant storage solution (commercially available CPDA, containing citrate/phosphate/dextrose/adenine), and suspended in dextrose-saline at a 10% hematocrit. Approximately 10 ml of washed packed red cells is placed in a quartz chamber and exposed to U.V. light, preferably at 320 nm, for 2 minute time intervals, up to a 10 minute total exposure. At each 2 minute interval the suspension is mixed and a small sample of red cells (10 microliters) is removed and diluted into 2 ml of water for spectrophotometric assay of hemoglobin. At each step the temperature of the irradiated red cell suspension is measured, to. ensure that the suspension did not overheat. At no point did the suspension exceed 26 degrees C (normal body temperature is 37 degrees C). Untreated red cells contain a high proportion of functional oxyhemoglobin (oxyHb), usually in the range of 96% or higher. Oxidation damage can form a semi-stable methemoglobin species (metHb), which can normally be reduced back to oxyhemoglobin by a cellular repair enzyme. Hemichrome represents a more severely damaged form, and can be irreversible. Normal red cells can tolerate a moderate level of methemoglobin. Hemichrome degradation can produce free heme, the iron-porphyrin component of native hemoglobin, which is damaging to cell membranes. Thus it is desirable to minimize hemichrome levels. Each hemoglobin species can be detected at a specific wavelength, using a standard spectrophotometer.

The following data show the sensitivity of the hemoglobin to damage by the increased U.V. exposure.

An exposure of 3 minutes was judged to be usable for viral inactivation using a radiation sensitizer, without inflicting excessive damage to red blood cells.

| EXPOSURE (Minutes) | % OXYHB | % METHB | % HEMI |
|---|---|---|---|
| 0 | 96.6 | 3.4 | 0 |
| 2 | 90.2 | 7.5 | 2.3 |
| 4 | 84.5 | 13.4 | 2.1 |
| 6 | 76.7 | 22.5 | 0.9 |
| 8 | 72.6 | 27.4 | 0 |
| 10 | 66.4 | 33.6 | 0 |

EXAMPLE 2

A suspension (0.1 ml) of bacteriophage lambda or bacteriophage phi-X174, of at least 10EV PFU/ml, is separately added to 4 ml of dextrose-saline containing 1 mg/ml of compounds I or II or III. Each suspension of bacteriophage with a radiation sensitizing compound is then exposed to U.V. radiation of the preferred wavelength (320 nm) in a quartz chamber for the preferred time (3 minutes). A control sample of each bacteriophage suspension, containing a sensitizer, is not exposed to U.V. light. Serial dilutions are performed to quantitate the level of infectious titer, and aliquots of the various bacteriophage samples are then mixed with host bacteria and spread on nutrient agar. Following a normal growth period, the plates are assayed for plaques. Other bacteriophage suspensions are separately irradiated as above, but without added sensitizer, to demonstrate the effect of this dose of U.V. alone.

| | Log10 Reduction of Virus Titer | |
|---|---|---|
| COMPOUND | phi-X174 | Lambda |
| I (X=$N_3$) | >6.0 | >6.0 |
| I (X=I) | 4.0 | >6.0 |
| II | 1.7 | >6.0 |
| No compound | 2-3 | 2-3 |

From these data it can be seen that all three tested compounds significantly increase the sensitivity of double-stranded DNA virus (lambda) to U.V. of the preferred exposure. Compound I is also effective against a single-stranded DNA virus, phi-X174. Compound I is most preferred, showing a high (at least 6 log reduction) inactivation efficacy against both single-strand and double-strand DNA viruses.

## Claims

1. A process for reducing viral and/or bacterial contamination in a composition comprising red blood cells, platelets and/or proteins, said process comprising the steps of contacting said composition with at least one chemical sensitizer selected from the group consisting of compounds which bind to DNA and/or RNA, and exposing said composition to radiation of sufficient wavelength and intensity for a period of time sufficient to cause said sensitizer to further reduce viral and bacterial contamination in said composition; **characterised by** lyophilizing the composition prior to said step of exposing said composition to radiation.

2. The process of claim 1, wherein said chemical sensitizer comprises a metal atom.

3. The process of claim 1, wherein said chemical sensitizer is selected from the group consisting of compounds of the formula:

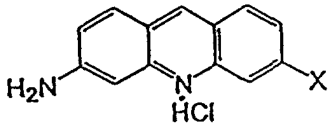

(I)

$X = -N_3, -I$

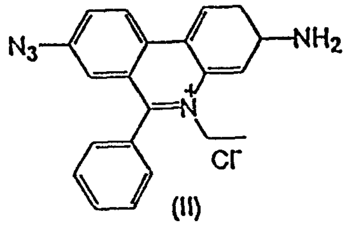

(II)

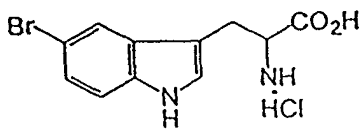

(III)

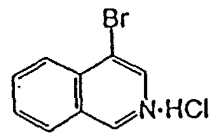

(IV)

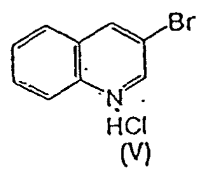

(V)

4. The process of any of claims 1-3, wherein said chemical sensitizer is sensitized by penetrating, ionizing radiation.

5. The process of claim 4, wherein said chemical sensitizer is sensitized by gamma radiation or X-rays.

**Patentansprüche**

1. Verfahren zur Verringerung der viralen und/oder bakteriellen Kontamination in einer rote Blutkörperchen, Blutplättchen und/oder Proteine enthaltenden Zusammensetzung, wobei das Verfahren folgende Stufen umfasst: man bringt die Zusammensetzung mit mindestens einem chemischen Sensibilisierungsmittel, das aus der Gruppe von Verbindungen, die DNA und/oder RNA binden, besteht, in Kontakt und setzt die Zusammensetzung einer Strahlung von ausreichender Wellenlänge und Intensität für eine ausreichende Zeitspanne aus, so dass das Sensibilisierungsmittel dazu veranlasst wird, in der Zusammensetzung eine weitere Verringerung der viralen und/oder bakteriellen Kontamination zu bewirken, **dadurch gekennzeichnet, dass** die Zusammensetzung vor der Stufe der Strahlungseinwirkung lyophilisiert wird.

2. Verfahren nach Anspruch 1, wobei der chemische Sensibilisator ein Metallatom umfasst.

3. Verfahren nach Anspruch 1, wobei der chemische Sensibilisator aus der Gruppe der Verbindungen der folgenden Formeln ausgewählt wird:

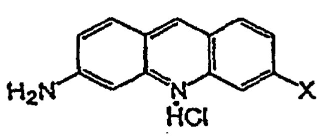

(I)

$X = -N_3, -I$

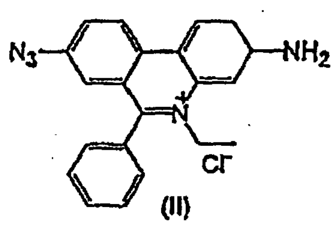

(II)

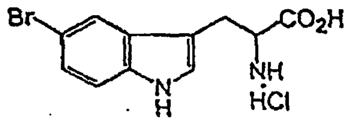

(III)

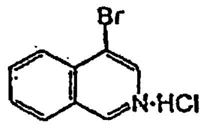

(IV)

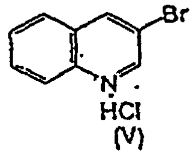

(V)

4. Verfahren nach Anspruch 1 bis 3, wobei der chemische Sensibilisator durch penetrierende, ionisierende Strahlung sensibilisiert wird.

5. Verfahren nach Anspruch 4, wobei der chemische Sensibilisator durch gamma-Strahlen oder Röntgenstrahlen sensibilisiert wird.

## Revendications

1. Procédé pour réduire la contamination virale et/ou bactérienne dans une composition comprenant des globules rouges, des plaquettes et/ou des protéines, ledit procédé comprenant les étapes consistant à mettre en contact ladite composition avec au moins un sensibilisant chimique choisi dans le groupe consistant en des composés qui se lient à l'ADN et/ou à l'ARN, et à exposer ladite composition à un rayonnement ayant une longueur d'onde et une intensité suffisantes pendant une période de temps suffisante pour amener ledit sensibilisant à réduire davantage la contamination virale et bactérienne dans ladite composition ; **caractérisé par** la lyophilisation de la composition avant ladite étape d'exposition de ladite composition à un rayonnement.

2. Procédé suivant la revendication 1, dans lequel ledit sensibilisant chimique comprend un atome de métal.

3. Procédé suivant la revendication 1, dans lequel ledit sensibilisant chimique est choisi dans le groupe consistant en des composés de formules :

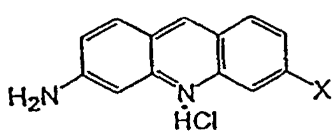

(I)

X = -$N_3$, -I

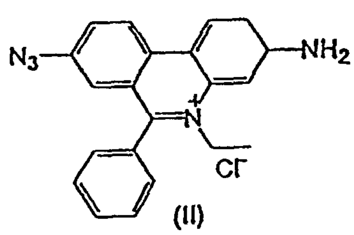

(II)

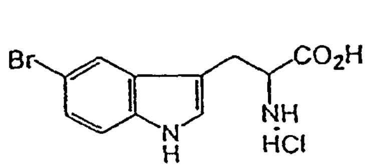

(III)

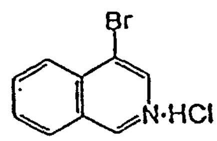

(IV)

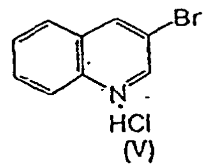

(V)

**4.** Procédé suivant l'une quelconque des revendications 1 à 3, dans lequel ledit sensibilisant chimique est sensibilisé par un rayonnement ionisant pénétrant.

**5.** Procédé suivant la revendication 4, dans lequel ledit sensibilisant chimique est sensibilisé par un rayonnement gamma ou des rayons X.